# EUROPEAN PATENT APPLICATION

(11) **EP 2 228 055 A1**
(43) Date of publication of application: **15.09.2010**
(21) Application number: 09425102.2
(22) Date of filing: 13.03.2009
(51) Int. Cl.: A61K 9/00, A61K 9/107, A61K 31/428, A61P 25/28, A61K 47/44, A61K 47/26

(54) **Riluzole liquid emulsions**

(71) Applicant: ITALFARMACO S.p.A., 20126 Milano (IT)
(72) Inventor: Artico, Roberta, 20126 Milano (IT); Adami, Marco, 20126 Milano (IT); Gianmillari, Salvatore Agostino, 20126 Milano (IT); Moscoso, Jaime, 28108 Alcobendas (Madrid) (ES); Oldoni, Tiziano, 20126 Milano (IT); Mascagni, Paolo, 20126 Milano (IT)
(74) Representative: Pistolesi, Roberto

(57) **Abstract**

Physically and chemically stable liquid O/W and W/O emulsions of riluzole are disclosed, together with methods for the preparation thereof. Such emulsions contain riluzole or a pharmaceutically acceptable salt or derivative thereof in amounts from about 0.1 % to about 20 % w/w, preferably from about 0.5% to about 5% w/w; such emulsions are devoided of the known local (mouth) anaesthetic effects of riluzole.

## Description

The present invention relates to physically and chemically stable liquid emulsions of riluzole for oral administration, having minimal or no anaesthetic effect in the mouth.

### BACKGROUND OF THE INVENTION

Riluzole (6-(trifluoromethoxy)benzothiazol-2-amine) is a compound of formula

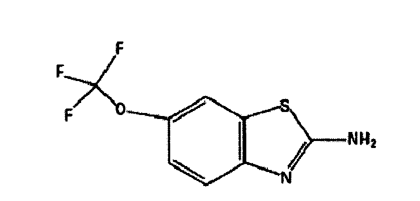

Riluzole, a drug used to treat amyotrophic lateral sclerosis (ALS, sometimes called Lou Gehrig's disease, Maladie de Charcot or motor neurone disease), is usually administered in an oral form at the dose of 50 mg every 12 hours and it delays the onset of ventilator-dependence or tracheostomy in selected patients.

ALS is a progressive, fatal neurodegenerative disease caused by the degeneration of motor neurons. It is marked by gradual degeneration of the central nervous system nerve cells that control voluntary muscle movement. As motor neurons degenerate, they can no longer send impulses to the muscle fibers that normally result in muscle movement. Early symptoms of ALS often include increasing muscle weakness, especially involving the arms and legs, speech, swallowing or breathing.

Riluzole is approved in the US and Europe for ALS disease.

US5527814 discloses the use of riluzole for treating ALS; the riluzole-based formulations exemplified in this patent are a tablet, a hard gelatine capsule and an injectable solution.

US6432992 discloses the use of riluzole for the treatment of adrenoleukodystrophy; also the riluzole-based formulations exemplified in this patent are a tablet, a hard gelatine capsule and an injectable solution.

Oral Riluzole is commercially available as capsule-shaped, white, film-coated tablets containing 50 mg active. Currently there are no commercially available liquid formulations of riluzole.

As opposed to tablets, an oral liquid formulation would increase ALS patients compliance when difficulties in swallowing arise.

Riluzole has a very low solubility in water, about 0.3 mg/mL at neutral pH. Although under acidic conditions the solubility of Riluzole increases and at pH 1.2 is about 12 mg/mL, its chemical stability decreases dramatically. So aqueous acidic solutions for oral use are not feasible.

Nevertheless, it is possible to prepare aqueous solutions having concentrations of riluzole between 0.25 and 10% w/w that are not only technically feasible (for example by using co-solvents or solubilizers to increase riluzole aqueous solubility) but also physically and chemically stable. These solutions, however, exhibit very poor palatability due to a significant and persistent (lasting more than 20-30 minutes) anesthetic effect in the mouth, which is due to the intrinsic property of the drug itself.

Due to its lipophilic character, riluzole can be easily solubilized in oils, and is therefore a good candidate for an emulsion formulation.

Emulsions are dispersed, two-phase systems, in which one phase ("internal" phase) is dispersed as droplets in the second phase ("continuous" or "external" phase). As such, they are by definition thermodynamically unstable and tend to revert to an energetically more stable state by for example undergoing aggregation, coalescence and creaming,.

An emulsion is a dispersed system containing at least two immiscible liquid phases (typically oily and aqueous phases). Emulsions are also thermodynamically unstable and the dispersed droplets tend to aggregate or coalesce. Therefore an emulsifying agent is added to stabilize the system. Nevertheless, which emulsifying agent will be successful in stabilizing the formulation is not predictable and its choice is critical to the physical stability of an emulsion.

### DESCRIPTION OF THE INVENTION

We have now surprisingly found that by using certain specific excipients, orally administerable liquid emulsions of riluzole are obtained, which have minimal or no anaesthetic effect at all in the mouth, thus resulting in improved patient compliance.

These liquid emulsions are physically and chemically stable, which is an essential requirement for industrial preparation and distribution of the corresponding pharmaceutical formulations.

On the other hand, the emulsions of the present invention are feasible in a wide range of riluzole concentrations, e.g., from 0.1 % to more than 15 % (w/w). This provides the doctors with different dosage and administration regimens; it permits to personalize the treatment and, therefore, improves patient compliance.

Accordingly, a first aspect of the invention relates to stable liquid emulsions of riluzole with minimal or no anaesthetic effect in the mouth. The emulsion of the present invention is thus preferably administered orally; nevertheless, such an emulsion may enable the enteric nutrition of those ALS patients, who, due to their reduced mobility, require additional routes of feeding.

In a second aspect the invention relates to said emulsions of riluzole for use in the treatment of ALS.

In a third aspect the invention relates to method(s) of preparation of these liquid emulsions of riluzole.

The emulsion of this invention can be either oil-in-water (O/W) where droplets of oil are dispersed in the aqueous phase, or water-in-oil (W/O), in which water is the dispersed (internal) phase and an oil is the dispersing medium. In both cases, riluzole will be preferentially dissolved in the oily phase.

Accordingly, the embodiment of the invention relates to physicochemically stable emulsions comprising riluzole or a pharmaceutically acceptable salt or derivative thereof dissolved in at least one oil.

Preferably, the amount of riluzole is between about 0.1% and about 20% w/w. In a more preferred embodiment, riluzole is present in an amount from about 0.2% to about 10% w/w, preferably from 0.3% to about 6%.

For the purposes of the present invention, the expression "w/w" is intended to indicate the weight of the mentioned compound with respect to the weight of the whole emulsion.

Any pharmaceutically acceptable oil can be used in the emulsions of the present invention.

Preferably, the oil is selected from the group of medium chain triglycerides (i.e. wherein glycerol is esterified with fatty acids having a chain length from 8 to 12 carbon atoms , such as caprylic-capric triglycerides); soybean oil; safflower oil; cottonseed oil; sesame oil; isopropyl myristate; ethyl oleate; olive oil; almond oil and mixtures thereof.

In a more preferred embodiment the oil is selected from caprylic-capric triglycerides, isopropyl myristate, sesame oil and/or soybean oil.

The oil is normally present in amounts from about 5 % to about 50 % w/w, more preferably in amounts from about 10 % to about 30 % w/w.

The emulsions of the present invention may also contain a surfactant, which may be of natural and/or synthetic origin.

Preferably the surfactant is present in an amount of between about 1 % and about 30 % w/w, more preferably of about 2.5 % and 10 % w/w.

Suitable natural surfactants for the present invention may be selected from gelatine, acacia, lecithins and/or cholesterol.

Suitable synthetic surfactants for the present invention may be selected from anionic, cationic and/or non-ionic surfactants.

Preferably, the surfactant is selected from the group of polyoxyethylene sorbitan fatty acid esters (or polysorbates, sold under Tween® trademark), sorbitan fatty acid esters (sold under Span® trademark), polyoxyethylene castor oil derivatives (sold under Cremophor® trademark), polyoxyethylene fatty ethers (or ethoxylated fatty alcohols, sold under Volpo® trademark), polyoxyethylene stearates and/or poloxamers.

According to a preferred embodiment of the invention, a mixture of two or more surfactants is used. In a more preferred embodiment, such a mixture is a mixture of a polysorbate and a sorbitan fatty acid.

Preferably the emulsions of the invention also include a so called "auxiliary" emulsifying agent, i.e. a compound which can help to stabilize the emulsion.

Preferred auxiliary emulsifying agents are selected from cetyl alcohol, stearic acid, glyceryl monostearate, sodium carboxymethyl-cellulose (NaCMC), hydroxypropylmethylcellulose (HPMC), methylcellulose (MC), hydroxyethylcellulose (HEC), natural gums and/or xanthan gum.

The emulsions of the invention may also include an anti-foaming agent (as for instance a simethicone emulsion).

The emulsions of the invention may also include a preservative.

The preservative may be any pharmaceutically acceptable antimicrobial agent. Preferably, it is selected from the group of methylparaben, ethylparaben, propylparaben, butylparaben, benzoic acid, sodium benzoate and mixtures thereof.

In a particularly preferred embodiment the preservative is a mixture of methyl and propylparaben.

The preservative is added in an amount enough to obtain an acceptable antimicrobial capacity. Preferably, it is in an amount between about 0.05 % and about 2 % w/w, more preferably between about 0.1 % and about 1.0 % w/w.

The emulsions of the present invention may also include at least one of the following excipients in amounts known by an skilled in the art:
- a sweetening agent;
- a flavouring agent;
- an anti-oxidant (as for instance α-tocopherol).

The emulsions according to the present invention can be prepared following any known process of the prior art. The present invention comprises therefore any method for manufacturing the emulsions of riluzole of the present invention.

In a particular embodiment an emulsion according to the present invention can be prepared according to the following steps:
(a) In a suitable vessel (for instance a jacketed stainless steel tank) a mixture of oil and surfactant(hereinafter referred to as the "oily phase") is heated to at least 65° - 70°C; the prescribed amounts of antioxidant (if required) and riluzole are added thereto and the mixture is stirred until dissolution. If required, the auxiliary emulsifying agent is added thereto and the mixture is stirred until dissolveld/dispersed.
(b) In a second vessel (e.g. a jacketed stainless steel tank) the aqueous phase is heated to at least 65° - 70°C; the preservative and the sweetener (if required) are added thereto and the mixture is stirred until dissolution. If required, the auxiliary emulsifying system is added thereto and the mixture is stirred until completely dispersed/dissolved.
(c) The aqueous phase is added to the oily phase under stirring, preferably a vigorous stirring agitation (for instance of ultraturrax-type).
(d) The mixture is kept under stirring while cooling to 30° - 45°C.
(e) The flavouring agent is added thereto under stirring while cooling to room temperature (i.e. to about 20-25° C).
(f) The emulsion thus-obtained is homogenized (for instance by using a colloid mill, piston-type and/or ultraturrax-type homogenizer).
(g) The thus-obtained emulsion is added into the containers (of glass or plastic) and capped.

According to an alternative embodiment, the order of addition of the two phases can be inverted (i.e., the oily phase may be added to the aqueous phase).

Possible pharmaceutical emulsions of the invention are provided in the attached examples which, however, are only intended to illustrate and not to limit the invention.

### EXAMPLES

For the purposes of the present invention the stability of liquid compositions of riluzole with excipients of different chemical nature, used alone or in combination, was measured.

The emulsions were prepared as reported above, using an ultraturrax-type homogenizer.

The physical stability of the emulsions was verified by the following techniques: appearance (by visual inspection) to verify the absence of coalescence of the internal phase and the absence of creaming; optical microscopy and laser diffraction (to determine droplet size distribution and verify whether or not drop size would increase).

The chemical stability of emulsions was assessed by means of a specific and stability indicating HPLC method.

The following examples are intended to illustrate the scope of the present invention in all its aspects but not to limit it there to.

### Example 1:

**Oral Emulsion - Riluzole 5 % (w/w)**

| *Ingredient* | *Quantity (mg)* |
|---|---|
| Riluzole | 50 |
| Methyl paraben | 1.35 |
| Propyl paraben | 0.15 |
| Ethyl oleate | 300 |
| Polysorbate 80 | 30 |
| Span 80 | 20 |
| Mint flavour | 7 |
| Purified water | q.s. to 1 g |

Stability: droplet size was less than 10 µm. The formulation was found to be physically stable for at least 30 days at room temperature.

### Example 2:

**Oral Emulsion - Riluzole 2.5 % (w/w)**

| *Ingredient* | *Quantity (mg)* |
|---|---|
| Riluzole | 25 |
| Methyl paraben | 1.35 |
| Propyl paraben | 0.15 |
| Caprylic-capric triglycerides (Miglyol 812N) | 200 |
| Polysorbate 80 | 40.7 |
| Span 80 | 9.3 |
| Sodium saccharine | 1 |
| Simethicone emulsion | 0.2 |
| Purified water | q.s. to 1 g |

Stability: droplet size was less than 10 µm. The formulation was found to be physically stable for at least 1 week at 40°C/75 % R.H.

### Example 3:

**Oral Emulsion - Riluzole 0.5 % (w/w)**

| *Ingredient* | *Quantity (mg)* |
|---|---|
| Riluzole | 5 |
| Methyl paraben | 1.35 |
| Propyl paraben | 0.15 |
| Ethyl oleate | 300 |
| Polysorbate 80 | 30 |
| Span 80 | 20 |
| Mint flavour | 7 |
| Purified water | q.s. to 1 g |

Stability: droplet size was less than 10 µm. The formulation was found to be physically stable for at least 30 days at room temperature.

### Example 4:

Palatability of riluzole emulsions (Example 1 and 3) was evaluated versus riluzole aqueous solutions containing parabens, mint flavor and polyoxyethylene castor oil derivative (Cremophor® RH40) as solubilizer.

Placebo was also included in the study, having the composition of Example 1, except for riluzole.

Samples of each composition were evaluated in blind by three scientists (herein referred to as "panelists") according to the following cross-over protocol:
- Samples were prepared by an independent scientists, thus panelists did not know what they were asked to taste.
- Each panelist was given 2 ml or 10 ml of each test product (corresponding to 50 mg of riluzole). The administered volume was swished in the mouth for about 5 seconds, then expelled from the mouth.
- A first evaluation was made during swishing, based on the general "mouth feel" (i.e., attributes such as sweetness, bitterness, astringent, etc.). An evaluation of the aftertaste was then made, based on mouth feeling, mouth irritation, and anaesthetic effects. An arbitrary four-point scale was used (0 through 3), with "0" meaning no anaesthetic effect and "3" indicating the strongest anaesthetic effect. The time required for the anaesthetic effect to disappear was recorded .
- Panelists were not allowed to use spring water to rinse their mouth for at least 30 minutes after expelling the sample.
- A 1 hour washout period was provided between samples. No more than 4 samples per day were evaluated by each panelist.

Results of the study can be summarized as follows:
- 5% riluzole emulsion: good general mouth feel, mild mouth irritation and anaesthetic effect (level 2), persisting for about 15 minutes.
- 5% riluzole solution: poor general mouth feel, with significant mouth irritation, lip burn and anaesthetic effect (level 3), persisting for at least 20-30 minutes, up to a maximum of 60 minutes (in one case).
- 0.5 % riluzole emulsion: good general mouth feel, no mouth irritation, very mild or no anaesthetic effect (level 0-1).
- 0.5 % riluzole solution: good general mouth feel, mild mouth irritation and anaesthetic effect (level 2), persisting for at least 15-20 minutes.
- Placebo: good general mouth feel, no anaesthetic effect (level 0).

These results represent a surprisingly favour palatability profile for the compositions of the invention by comparison with riluzole solution, indicating a significant advance in the art, especially in providing an effective treatment for amyotrophic lateral sclerosis with potential for a high degree of patient compliance.

## Claims

1. An emulsion containing riluzole or a pharmaceutically acceptable salt or derivative thereof in amounts from about 0.1 % to about 20 % w/w, preferably from about 0.2% to about 10% w/w, more preferably from about 0.3% to about 6% w/w.

2. An emulsion according to claim 1, **characterized by** containing an oil in amounts from about 5 % to about 50 % w/w, preferably from about 10 % to about 30 % w/w.

3. An emulsion according to claim 2, **characterized in that** said oil is selected from medium chain triglycerides, such as caprylic-capric triglycerides; soybean oil; safflower oil; cottonseed oil; sesame oil; isopropyl myristate; ethyl oleate; olive oil; almond oil and mixtures thereof.

4. An emulsion according to claim 2, **characterized in that** said oil is selected from caprylic-capric triglycerides, isopropyl myristate, sesame oil and/or soybean oil.

5. An emulsion according to claim 1, **characterized by** containing a surfactant in amounts from about 1 % to about 30 % w/w, preferably from about 2.5 % to about 10 % w/w.

6. An emulsion according to claim 5, **characterized in that** said surfactant is of natural origin and it is preferably selected from gelatine, acacia, lecithins and/or cholesterol.

7. An emulsion according to claim 5, **characterized in that** said surfacrtant is of synthetic origin and it is preferably selected from anionic, cationic and/or non-ionic surfactants.

8. An emulsion according to claim 7, **characterized in that** said surfactant is selected from polyoxyethylene sorbitan fatty acid esters, sorbitan fatty acid esters, polyoxyethylene castor oil derivatives, polyoxyethylene fatty ethers, polyoxyethylene stearates and/or poloxamers.

9. An emulsion according to claim 5, **characterized in that** said surfactant is a mixture of two or more surfactants, preferably a mixture of a polysorbate and a sorbitan fatty acid.

10. An emulsion acording to claim 1, **characterized by** containing cetyl alcohol, stearic acid, glyceryl monostearate, sodium carboxymethyl-cellulose (NaCMC), hydroxypropylmethylcellulose (HPMC), methylcellulose (MC), hydroxyethylcellulose (HEC), natural gums and/or xanthan gum.

11. An emulsion according to claim 1, **characterized by** containing an anti-foaming agent and/or a preservative.

12. An emulsion according to claim 1, **characterized in that** said preservative is selected from methylparaben, ethylparaben, propylparaben, butylparaben, benzoic acid, sodium benzoate and mixtures thereof, preferably it is a mixture of methyl and propylparaben.

13. An emulsion according to claim 1, **characterized in that** said preservative is present in amounts from about 0.05 % to about 2 % w/w, more preferably from about 0.1 % to about 1.0 % w/w.

14. An emulsion according to claim 1, **characterized by** containing a sweetening agent, a flavouring agent and/or an anti-oxidant

15. An emulsion according to any of the preceding claims, for use in the treatment of amyotrophic lateral sclerosis.
